(19) Europäisches Patentamt / European Patent Office / Office européen des brevets

(11) **EP 4 196 006 B1**

(12) **EUROPEAN PATENT SPECIFICATION**

(45) Date of publication and mention of the grant of the patent:
**02.10.2024 Bulletin 2024/40**

(21) Application number: **21758964.7**

(22) Date of filing: **03.08.2021**

(51) International Patent Classification (IPC):
**A61B 5/01** (2006.01)    **A61B 5/00** (2006.01)
**A61B 5/0205** (2006.01)    **A61B 5/11** (2006.01)

(52) Cooperative Patent Classification (CPC):
**A61B 5/01; A61B 5/02055; A61B 5/681;**
**A61B 5/7264; A61B 5/7282; A61B 5/742;**
A61B 5/1112; A61B 5/4809; A61B 5/4842;
A61B 5/4857; A61B 5/7475

(86) International application number:
**PCT/US2021/044344**

(87) International publication number:
**WO 2022/035646 (17.02.2022 Gazette 2022/07)**

(54) **DETECTION OF USER TEMPERATURE AND ASSESSMENT OF PHYSIOLOGICAL SYMPTOMS WITH RESPIRATORY DISEASES**

ERKENNUNG DER BENUTZERTEMPERATUR UND BEURTEILUNG PHYSIOLOGISCHER SYMPTOME MIT ATEMWEGSERKRANKUNGEN

DÉTECTION DE TEMPÉRATURE D'UTILISATEUR ET ÉVALUATION DES SYMPTÔMES PHYSIOLOGIQUES EN CAS DE MALADIES RESPIRATOIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.08.2020 US 202063065254 P**

(43) Date of publication of application:
**21.06.2023 Bulletin 2023/25**

(73) Proprietor: **Fitbit LLC**
**San Francisco, CA 94105 (US)**

(72) Inventors:
• **LAFON, Belen**
**San Francisco, California 94105 (US)**
• **DESHPANDE, Aniket Sanjay**
**San Francisco, California 94105 (US)**
• **ZHANG, Xi**
**San Francisco, California 94105 (US)**
• **SUNDEN, Lindsey Michelle**
**San Francisco, California 94105 (US)**
• **NATARAJAN, Aravind**
**San Francisco, California 94105 (US)**
• **HENEGHAN, Conor Joseph**
**San Francisco, California 94105 (US)**

• **SU, Hao-Wei**
**San Francisco, California 94105 (US)**

(74) Representative: **Maikowski & Ninnemann**
**Patentanwälte Partnerschaft mbB**
**Postfach 15 09 20**
**10671 Berlin (DE)**

(56) References cited:
**WO-A1-2019/084312**    **CN-A- 107 049 253**
**US-A1- 2016 331 244**    **US-A1- 2019 323 895**

• **SARSENBAYEVA ZHANNA ZSARSENBAYEV@STUDENT UNIMELB EDU AU ET AL: "Sensing Cold-Induced Situational Impairments in Mobile Interaction Using Battery Temperature", PROCEEDINGS OF THE ACM ON INTERACTIVE, MOBILE, WEARABLE AND UBIQUITOUS TECHNOLOGIES, ACMPUB27, NEW YORK, NY, USA, vol. 1, no. 3, 11 September 2017 (2017-09-11), pages 1 - 9, XP058484931, DOI: 10.1145/3130963**

**(Cont. next page)**

• **LUO LAN ET AL: "Detection and Prediction of Ovulation From Body Temperature Measured by an In-Ear Wearable Thermometer", IEEE TRANSACTIONS ON BIOMEDICAL ENGINEERING, IEEE, USA, vol. 67, no. 2, 14 May 2019 (2019-05-14), pages 512 - 522, XP011767231, ISSN: 0018-9294, [retrieved on 20200120], DOI: 10.1109/TBME.2019.2916823**

**Description**

RELATED APPLICATIONS

This application claims the benefit of U.S. Provisional Patent Application No.: 63/065,254, filed on August 13, 2020.

BACKGROUND

Wearable electronic devices have gained popularity among consumers. A wearable electronic device may track a user's activities or biometric data using a variety of sensors. Data captured from these sensors can be analyzed in order to provide a user with information, such as an estimation of how far they walked in a day, their heart rate, how much time they spent sleeping, and the like. However, a technical problem exists relating to the ability to collect sufficient data to provide a user with an overall picture of their present or anticipated future health. In particular, conventional devices are limited in being able to detect parameters that accurately and automatically detect user temperature (as well as various other physical parameters) for assessment of physiological symptoms associated with respiratory diseases and/or other medical conditions. WO 2019/084312 A1 relates to a worksite safety system and method. The system uses wearable sensors to collect biometric data from workers. The data are used to compute a worker's core body temperature on the basis of an individual profile associating historical biometric data with measured core body temperature. If the computed core body temperature crosses certain thresholds, alert actions are performed. US 2016/331244 A1 describes a system and method for determining a core body temperature or a physiological function. The system and method include using multiple sensors in combination with analytic techniques.

BRIEF DESCRIPTION OF THE DRAWINGS

Various embodiments in accordance with the present disclosure will be described with reference to the drawings, in which:

FIG. 1 illustrates an example of a user with a wearable device on an extremity, in accordance with various embodiments of the present disclosure.
FIG. 2 illustrates an example of a user interacting with a wearable device on an extremity, in accordance with embodiments of the present disclosure.
FIG. 3 illustrates an example set of devices that are able to communicate, in accordance with embodiments of the present disclosure.
FIGS. 4A-4D are graphical representations of temperature responses, in accordance with embodiments of the present disclosure.
FIG. 5 is a graphical representation of a temperature response, in accordance with embodiments of the present disclosure.
FIG. 6 is a flow chart of an embodiment of a data processing system, in accordance with embodiments of the present disclosure.
FIG. 7 is a graphical representation of a temperature comparison, in accordance with embodiments of the present disclosure.
FIG. 8 is a flow chart of an embodiment of a method for determining a temperature change for a proxy temperature, in accordance with embodiments of the present disclosure.
FIG. 9 is a framework for assessing the presence of an illness, in accordance with embodiments of the present disclosure.
FIG. 10 is a visual representation of a series of input images representing user symptoms, in accordance with embodiments of the present disclosure.
FIG. 11 is a graphical representation of an illness predictor, in accordance with embodiments of the present disclosure.
FIG. 12 is a flow chart of an embodiment of a method for predicting an illness, in accordance with embodiments of the present disclosure.
FIGS. 13A-13D are graphical representations of calculated Z-scores for different data components, in accordance with embodiments of the present disclosure.
FIG. 14 illustrates a set of basic computer components of one or more devices of the present disclosure, in accordance with various embodiments of the present disclosure.

BRIEF DESCRIPTION

The invention is set forth in the independent claim. Specific embodiments are presented in the dependent claims.

In an unclaimed aspect, the present disclosure is directed to a method for assessing the presence of or likelihood of developing a medical condition of a user of a wearable computing device. The method includes receiving, from a first sensor on the wearable computing device, first temperature data relating to a first temperature measurement of the user. The method also includes receiving, from a second sensor on the wearable computing device, second temperature data relating to a second temperature measurement of the user, the second sensor being in a different location from the first sensor. Further, the method includes determining, based at least in part on the first temperature data and the second temperature data, a proxy temperature. Moreover, the method includes determining a temperature change, based at least in part on the proxy temperature. In addition, the method includes comparing the temperature change to a temperature threshold. Further, the method includes determining, via the wearable computing device, a preliminary assessment of the medical condition for the user based on the temperature change. Thus, the method includes generating and displaying, via a display of the wearable computing device, a recommendation for the user based on the preliminary assessment.

The first temperature data is a skin temperature of the user of the wearable computing device. The second temperature data is an internal temperature of the wearable computing device. Further, in an embodiment, the proxy temperature is correlated to a core body temperature of the user of the wearable computing device.

In additional embodiments, the temperature threshold is at least one of a standard deviation away from a baseline temperature or a specified temperature.

In further embodiments, the method includes receiving third temperature data, corresponding to the proxy temperature over a period of time and determining, based at least in part on the third temperature data, a baseline temperature.

In another embodiment, the method includes determining the preliminary assessment of the medical condition for the user based on the temperature change using at least one machine learning algorithm. In certain embodiments, the medical condition may include at least one of fever, illness, an ovulation event, or circadian rhythm fluctuations.

In yet another unclaimed aspect, the present disclosure is directed to a method for assessing the presence of or likelihood of developing an medical condition of a user of a wearable computing device. The method includes receiving, from one or more sensors on the wearable computing device, first data indicative of a physiological response of a user. The method also includes receiving, as an input from the user of the wearable computing device, second data indicative of at least one of demographic information or health information relating to the user. Further, the method includes determining, using a trained neural network of the wearable computing device, a Z-score for at least one component of the first data. Moreover, the method includes determining, using a trained machine learning system of the wearable computing device, a probability that a severity of the at least one component exceeds a threshold. In addition, the method includes providing, via the wearable computing device to the user, an indication to perform an action.

In an embodiment, the first data is at least one of temperature, respiratory rate, oxygen, heart rate variability, or blood pressure waveform changes of the user. In another embodiment, the demographic information is at least one of age, sex, or geographic location of the user, and the health information is at least one of one or more symptoms, BMI, or co-morbidities of the user. In further embodiments, the severity corresponds to a likelihood of a requirement of medical intervention. No methods by themselves form part of the claims.

DETAILED DESCRIPTION

In the following description, various embodiments will be described. For purposes of explanation, specific configurations and details are set forth in order to provide a thorough understanding of the embodiments. However, it will also be apparent to one skilled in the art that the embodiments may be practiced without the specific details. Furthermore, well-known features may be omitted or simplified in order not to obscure the embodiment being described.

As computing devices become more ubiquitous and portable, many advantages are being seen in the field of health monitoring and diagnostics. Computing devices, particularly ones that can be worn or carried by a user, may include one or more sensors to detect physiological information about the user and/or the environment around the user. This information can be used to observe, detect, or diagnose various health conditions outside of a traditional clinic or laboratory setting. For example, in the context of detecting illness and/or various medical conditions, portable or wearable electronic devices may be able to detect changes in temperature throughout the user's body. Additionally, a computing device may be able to record and interpret the detected information about the user and/or environment, to determine a health assessment. As in the previous example, a wearable electronic device may be able to record changes in core body temperature and generate an assessment that the user is experiencing an illness or medical condition.

Thus, the present disclosure is directed to a technical solution/benefit to the aforementioned technical problem relating to the ability to collect sufficient data to provide a user with an overall picture of their present or anticipated future health. Accordingly, the present disclosure is directed to a system and method for assessing the presence of or likelihood of developing a medical condition of a user of a wearable computing device. In particular embodiments, for example, the wearable computing device includes first and second sensors for generating first and second temperature data from different locations. A proxy temperature can then be determined based on the first and second temperature data such that a temperature change can be determined and compared to a threshold. Changes above the threshold can thus be used to determine a preliminary assessment of the medical condition for the user based on the temperature change. The wearable computing device can then generate and display a recommendation for the user based on the preliminary assessment. Thus, the system and method of the present disclosure provide the technical benefit of early illness detection that can be useful in preventing and reducing the spread of illness.

In various embodiments, the wearable device may include a watch or ring that may be used as a proxy for a user's body temperature (e.g., basal temperature, core body temperature, etc.). For example, a first temperature may be taken at the wrist and a second temperature may be taken internal to the device, for example, using one or more sensors within the device. A correlation between the skin temperature and the internal device may enable an estimation of the user's body temperature.

Additionally, the wearable devices, which may be communicatively coupled to one or more other devices, may also receive user input regarding potential symptoms experienced by the user. These symptoms may then be collected and evaluated against sensor information, such as user temperature, respiratory rate, oxygen, heart rate variability, and the like. An evaluation may be performed to provide a preliminary diagnosis and/or recommendation to seek a diagnosis for a potential illness, such as a respiratory illness. Furthermore, in various embodiments, data may be utilized to predict an upcoming illness and/or predict a severity of an illness.

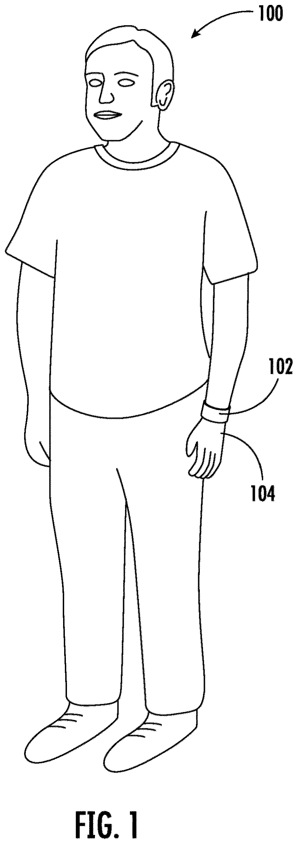

Referring now to the drawings, FIG. 1 illustrates an example of a user 100 wearing a wearable computing device 102 around a wrist 104 of the user 100. The wearable computing device 102 may also be referred to as a wearable or a fitness tracker, and may also include devices that are worn around the chest, legs, head, or other body part, or a device to be clipped or otherwise attached onto an article of clothing worn by the user 100. The wearable computing device 102 may collectively or respectively capture data related to any one or more of caloric energy expenditure, floors climbed or descended, heart rate, heart rate variability, heart rate recovery, location and/or heading (e.g., through GPS), elevation, ambulatory speed and/or distance traveled, swimming lap count, bicycle distance and/or speed, blood pressure, blood glucose, skin conduction, skin and/or body temperature, electromyography data, electroencephalographic data, weight, body fat, respiration rate and patterns, various body movements, among others. Additional data may be provided from an external source, e.g., the user may input their height, weight, age, stride, or other data in a user profile on a fitness-tracking website or application and such information may be used in combination with some of the above-described data to make certain evaluation or in determining user behaviors, such as the distance traveled or calories burned of the user. The wearable computing device 102 may also measure or calculate metrics related to the environment around the user such as barometric pressure, weather conditions, light exposure, noise exposure, and magnetic field.

In some embodiments, the wearable computing device 102 may be connected to a network directly, or via an intermediary device. For example, the wearable computing device 102 may be connected to the intermediary device via a BLUETOOTH® connection, and the intermediary device may be connected to the network via an Internet connection. In various embodiments, a user may be associated with a user account, and the user account may be associated with (i.e., signed onto) a plurality of different networked devices. In some embodiments, additional devices may provide any of the abovementioned data among other data, and/or receive the data for various processing or analysis. The additional devices may include a computer, a server, a handheld device, a temperature regulation device, or a vehicle, among others.

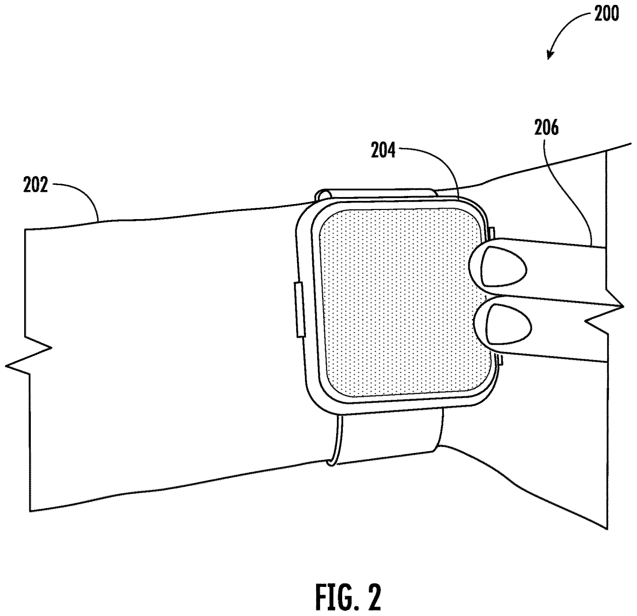

FIG. 2 illustrates an example wearable computing device 200 that can be utilized in accordance with various embodiments. In this example, the wearable computing device 200 is a smart watch, although fitness trackers and other types of devices can be utilized as well. Further, although the wearable computing device 200 is shown to be worn on a user's wrist, similar to the example of FIG. 1, there can be other types of devices worn on, or proximate to, other portions of a user's body as well, such as on a finger, in an ear, around a chest, etc. For many of these devices there will be at least some amount of wireless connectivity, enabling data transfer between a networked device or computing device and the wearable device. This might take the form of a BLUETOOTH® connection enabling specified data to be synchronized between a user computing device and the wearable device, or a cellular or Wi-Fi connection enabling data to be transmitted across at least one network such as the Internet or a cellular network, among other such options.

As mentioned, there can be various other types of functionality offered by such a wearable device, as may relate to the health of a person wearing the device. By way of example, there may be sensors embedded within the device that can collect data, which may be processed on the device, on a connected device, at a remote server, or some combination thereof. Further, as shown, the wearable computing device 200 of FIG. 2 is positioned on an arm 202 of a user. The wearable computing device 200 includes a screen 204 that the user can interact with, for example by inputting information using their fingers 206. For example, the screen 204 may prompt the user to answer one or more questions. Additionally, as noted herein, the screen 204 may also provide additional information and/or show connected devices that may also be used to input information that may be used by the device 200.

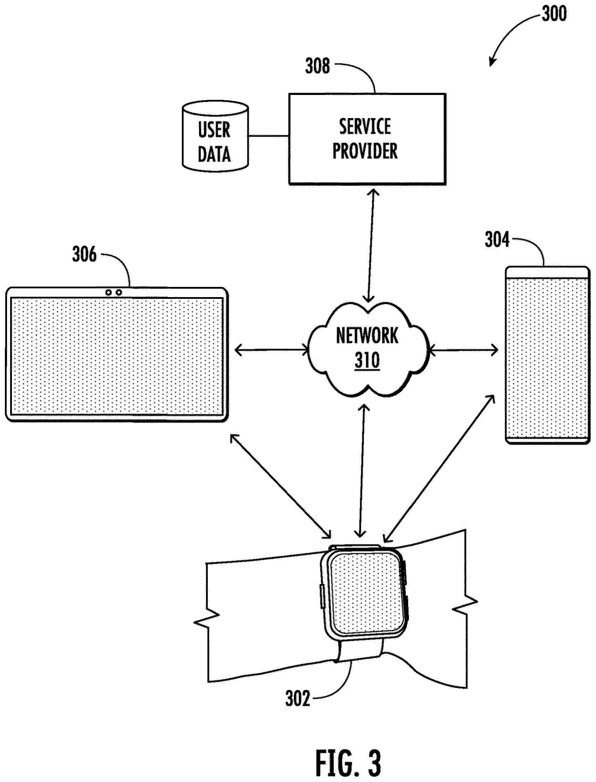

FIG. 3 illustrates an example environment 300 in which aspects of various embodiments can be implemented. In this example, a person might have a number of different devices that are able to communicate using at least one wireless communication protocol. In this example, the user might have a smart watch 302 or fitness tracker, which the user would like to be able to communicate with a smart phone 304 and a tablet computer 306. The ability to communicate with multiple devices can enable a user to obtain information from the smart watch 302, such as heart rate data captured using a sensor on the smart watch, using an application installed on either the smart phone 304 or the tablet 306. The user may also want the smart watch 302 to be able to communicate with a service provider 308, or other such entity, that is able to obtain and process data from the smart watch and provide functionality that may not otherwise be available on the smart watch or the applications installed on the individual devices. The smart watch may be able to communicate with the service provider 308 through at least one network 310, such as the Internet or a cellular network, or may communicate over a wireless connection such as Bluetooth® to one of the individual devices, which can then communicate over the at least one network. There may be a number of other types of, or reasons for, communications in various embodiments.

In addition to simply being able to communicate, a user may also want the devices to be able to communicate in a number of ways or with certain aspects. For example, the user may want communications between the devices to be secure, particularly where the data may include personal health data or other such communications. The device or application providers may also be required to secure this information in at least some situations. The user may want the devices to be able to communicate with each other concurrently, rather than sequentially. This may be particularly true where pairing may be required, as the user may prefer that each device be paired at most once, or that not manual pairing is required. The user may also desire the communications to be as standards-based as possible, not only so that little manual intervention is required on the part of the user but also so that the devices can communicate with as many other types of devices as possible, which is often not the case for various proprietary formats. A user may thus desire to be able to walk in a room with one device and have the device automatically communicate with another target device with little to no effort on the part of the user. In various conventional approaches, a device will utilize a communication technology such as Wi-Fi to communicate with other devices using wireless local area networking (WLAN). Smaller or lower capacity devices, such as many Internet of Things (IoT) devices, instead utilize a communication technology such as Bluetooth®, and in particular Bluetooth Low Energy (BLE) that has very low power consumption.

An environment 300 such as that illustrated in FIG. 3 enables data to be captured, processed, and displayed in a number of different ways. For example, data may be captured using sensors on the smart watch 302, but due to limited resources on that smart watch the data may be transferred to the smart phone 304 or service provider system 308 (or a cloud resource) for processing, and results of that processing may then be presented back to that user on the smart watch 302, the smart phone 304, or another such device associated with that user, such as the tablet computer 306. In at least some embodiments, a user may also be able to provide input such as health data using an interface on any of these devices, which can then be considered when making that determination.

In at least one embodiment, data determined for a user can be used to determine state information, such as may relate to a current arousal level or state of that user. At least some of this data can be determined using sensors or components able to measure or detect aspects of a user, while other data may be manually input by that user or otherwise obtained. In at least one embodiment, an arousals determination algorithm can be utilized that takes as input a number of different inputs, where different inputs can be obtained manually, automatically, or otherwise. In at least one embodiment, such an algorithm can take various types of factors identify events or activations related to arousal or "stress" events that activate a sympathetic nervous system response.

It may be challenging to determine a user's core body temperature based on a measurement from an extremity, such as an arm or a leg. For example, in certain situations, a user's physiological response may be to direct blood toward their core. Additionally, certain users may have "cold hands" regardless of other factors. Accordingly, traditional skin temperature measurements at a user's extremity have provided low accuracy for determining a user's core body temperature, which may be evaluated to identify a user with a fever. Embodiments of the present disclosure are directed toward using a combination of temperature measurements, such as a user's skin temperature and an internal device temperature, to serve as a proxy for core body temperature. Core body temperature may be used to determine various physical conditions of a user, such as fever, ovulation events, circadian rhythm fluctuations, and the like.

Embodiments of the present disclosure may use one or more machine learning systems to evaluate a user's temperature fluctuations to determine wrist and/or device temperature as a proxy for body temperature. For example, temperatures at the wrist may be logged over a period of time (e.g., every hour, every half hour, etc.). It should be appreciated that the period for logging temperature may be at night where there is less anticipated change due to environmental factors, such as sun exposure, activity levels, and the like.

Additionally, information may be obtained from an internal temperature sensor (e.g., internal to the device). In various embodiments, for example, where this is good contact between the user and the device, the temperatures may be substantially similar. The internal temperature may also be collected over a period of time, which may correspond with the period of time for collecting the user wrist, or skin, temperature.

Various embodiments of the present disclosure may also include user information that may serve as "ground truth" data for a machine learning system to evaluate a user's overall health levels. For example, the user may manually report feeling warm or hot during the day, such as through an application on a smart phone or via prompted questions from the device. Accordingly, the temperature sensors and/or correlation formed by the temperature sensors may be informed by the user's self-reported information. Thereafter, the user's temperature may be monitored over a period of time, such as days, to detect an increase or a decrease in temperature. For example, the user may report they are starting to feel unwell. The device may analyze temperature trends over the preceding days and detect a temperature increase over some period of time. Thereafter, the device may continue to monitor temperature and inform the user when their temperature exceeds a threshold, which may be indicative of a fever, or may alert the user that their temperature has exceeded a certain change threshold over a period of time.

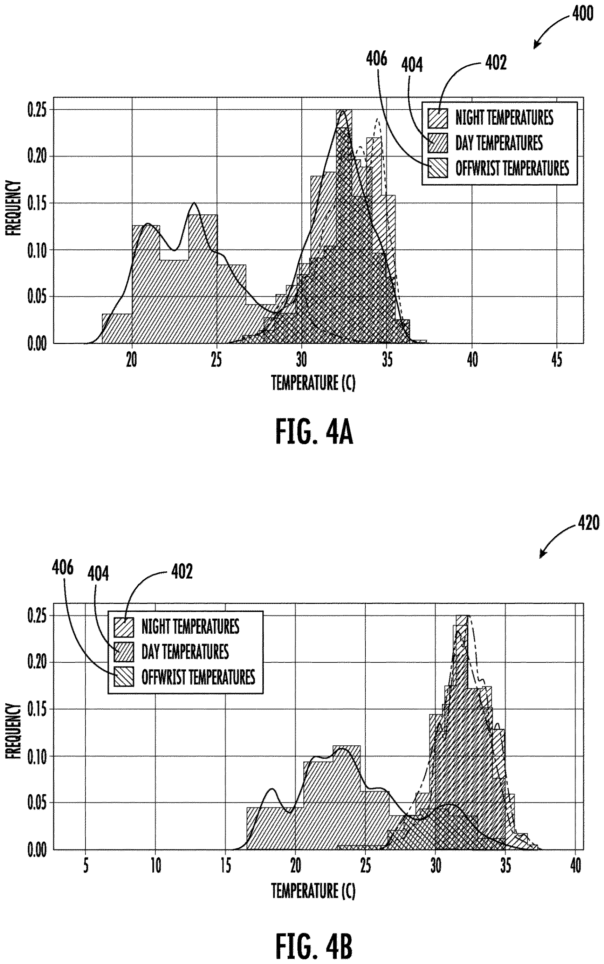

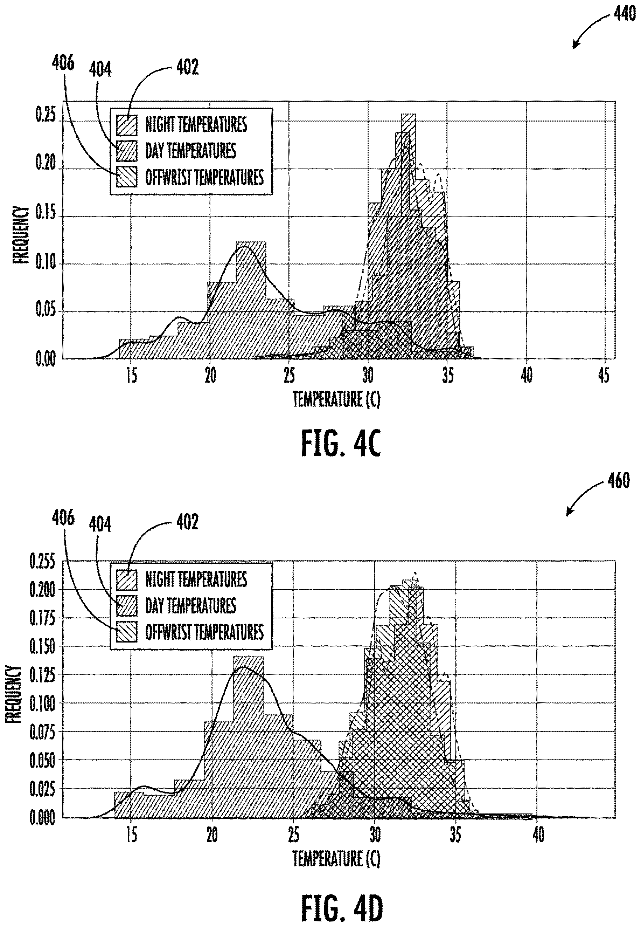

As noted above, in various embodiments, temperature may be collected during the night, such as when the user is asleep, due to reduced activity levels that may otherwise change or affect the user's body temperature. FIGS. 4A-4D are graphical representations 400, 420, 440, 460 of temperature distributions for night temperatures 402, day temperatures 404, and off-wrist temperatures 406. In the illustrated embodiments, the off wrist temperature 406 is shown to have a distribution that is less than both the night temperatures 402 and the day temperatures 404. Accordingly, this information may be useful in predicting when the user is not wearing the device, thereby removing the data for temperature measurements, which may artificially reduce the user's temperature information. Moreover, such measurements indicate the user is supplying heat to the device. Each of FIGS. 4A-4D illustrate a correlation between the night temperature 402 and the day temperature 404. Accordingly, it is likely that the external factors may not significantly affect the internal temperature of the device.

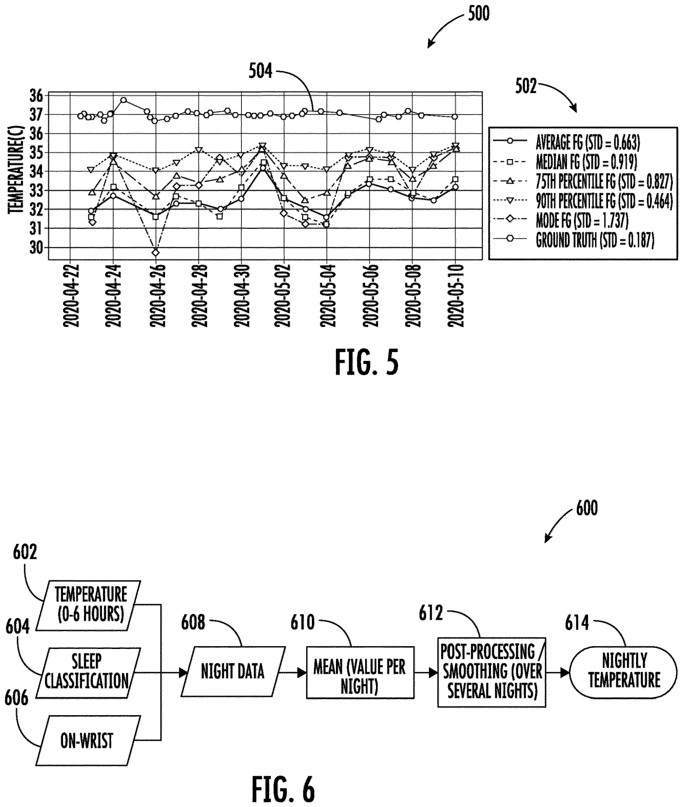

FIG. 5 is a graphical representation 500 of temperature measurements taken over a period of days compared to a ground truth. The temperature measurements may correspond to an internal device temperature, which may be provided by a temperature sensor for a related internal component, such as a sensor on the battery, thereby simplifying the device by reducing a number of components added to the interior chamber. In this example, different readings are evaluated, including average, median, 75$^{th}$ percentile, 90$^{th}$ percentile, and mode. This collection of readings 502 is compared against a ground truth 504. In the illustrated embodiment, the mean temperature was found to provide data substantially similar to the other candidates, and for simplicity, may be utilized with embodiments of the present disclosure to track temperature changes over time.

FIG. 6 is a flow chart of an embodiment of a process 600 for smoothing or processing data in order to determine a nightly temperature. In the illustrated embodiment, the process receives, as input data, temperature data 602, a sleep classification 604, and on-wrist data 606. The temperature data 602 may correspond to readings, which may be taken over a period of time and may be further sampled and smoothed over sub-periods of time. The sleep classification 604 may be a score or indication of the user's sleep state, such as the user being asleep, awake, etc. The on-wrist data 606 may provide information to determine which periods of time the device was on the user's wrist. Periods of time where the device was not on wrist may be discarded because they may provide artificially low temperatures, as shown in FIGS. 4A-4D.

In this example, nighttime data 608 is extracted from the input information 602, 604, 606 and a mean value may be calculated, as shown at 610. Post-processing and/or smoothing operations 612 may also be performed to determine the nightly temperature 614. For example, post-processing may include removing outlier information, changing a period of time for evaluation, and the like. Post-processing may be option depending on noise, data acquisition errors, and the like. Embodiments enable a calculation of a baseline temperature for a user. The baseline temperature is the average of nightly temperature values calculated over several nights. The baseline temperature should not fluctuate very much due to physiologically relevant events such as fever or ovulation. In various embodiments, the baseline temperature is calculated by averaging four (4) weeks of nightly temperature values. However, if less than four weeks of data is available, the baseline may be established using less time (e.g., two or three nights) and then updated as more data is made available.

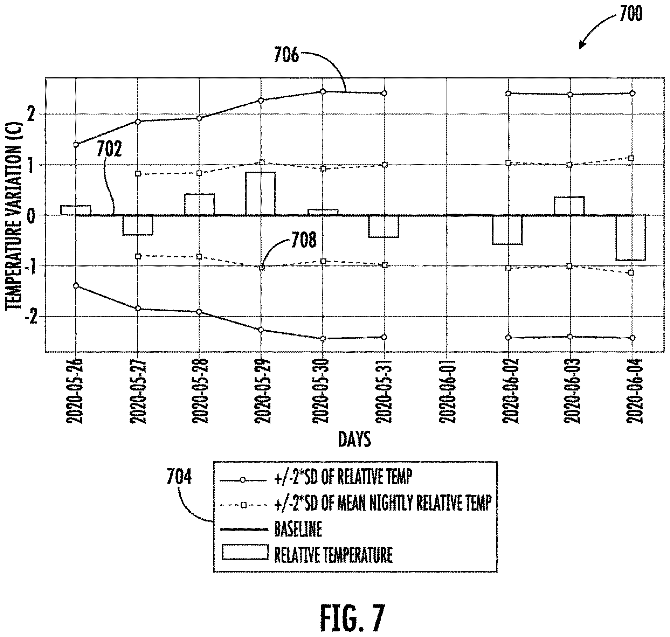

Once baseline information is established, changes in temperature may then be evaluated against a threshold in order to determine a physiologically relevant event. By way of example, the baseline may correspond to a median sleeping temperature across several nights, a relative temperature may be a difference between a nightly mean temperature and the baseline, an elevated temperature may be a classified value (e.g., two standard deviations from baseline or greater than 36 C), and a lowered temperature may also be a classified value (e.g., less than 2 standard deviations from baseline. FIG. 7 is a graphical representation 700 of a temperature analysis over a period of time. As shown, a baseline 702 is provided at the zero location and relative temperatures 704 as shown as deviations from the baseline. Both elevated and lowered markers 706, 708 are provided at two standard deviations from baseline for the relative temperature and of mean nightly relative temperature. Accordingly, information may be evaluated to determine an elevated temperature that may be indicative of fever, ovulation, or the like.

Data sources have different resolutions may be utilized with embodiments of the present disclosure. By way of example only, a first data set may record min and max temperature every hour. The first data set may have a 0.01 degrees Celsius (°C) precision. The average of these minimum and maximum values can be used as a proxy for true mean. As another example, a second data set may record temperature ever minute. The second data set may have a 0.1°C precision. It should be appreciated that, for lower resolution data, a correlation may be calculated in order to use either of the data sets.

It should be appreciated that a variety of different sensor data may also be incorporated into embodiments of the present disclosure in order to evaluate temperature data. By way of example, information from a UV sensor or light sensor may determine whether it is day or night and/or whether the wearable is beneath a cover or blanket or on the outside. Furthermore, a pressure sensor may be integrated into the wearable device to determine whether contact with the user's wrist is sufficient to obtain a skin temperature measurement. Additionally, a GPS signal may provide a location for the user to determine whether they are outside or undergoing physical activity. Accordingly, a variety of different pieces of sensor data may be utilized with embodiments of the present disclosure in order to determine whether one or more of the skin temperature and/or internal device temperature may serve as a proxy for body temperature and determination of changes in body temperature.

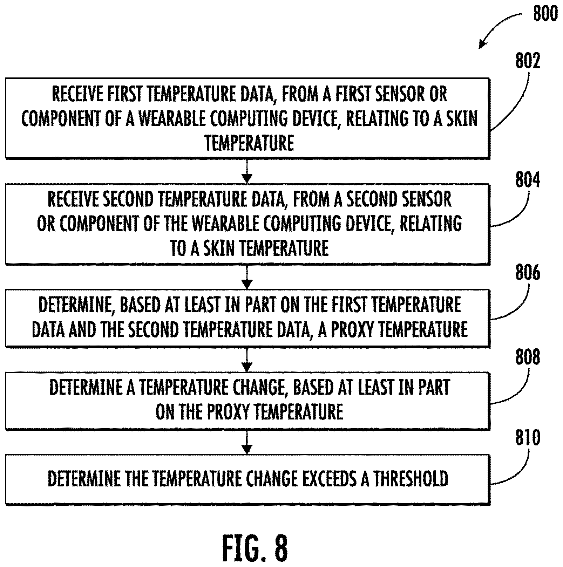

FIG. 8 is a flow chart of an embodiment of a method 800 for determining a proxy temperature according to the present disclosure. It should be understood that, for any process discussed herein, there can be additional, fewer, or alternative steps performed in similar or alternative orders, or in parallel, within the scope of the various embodiments. In this example, as shown at 802, a first temperature data is received from a first sensor or component of a wearable computing device. The first temperature data may be indicative of a skin temperature, such as a skin temperature at a wrist of a user wearing the wearable computing device. In an embodiment, for example, the skin temperature may be a contact sensor that is pressed against the skin to obtain a surface temperature of the skin. As noted above, the skin temperature may be sampled over a period of time, averaged, or the like and, in certain embodiments, may also correspond to an average temperature over a period of time.

As shown at 804, second temperature data may be received from a second sensor or component of the wearable device. According to the invention, the second temperature data corresponds to an internal device temperature for the wearable computing device. For example, the internal device temperature may be obtained from one or more sensors associated with components of the device, such as a sensor measuring a battery temperature or the like. As shown at 806, the combination of at least the first temperature data and the second temperature data is used to determine a proxy temperature. By way of example, changes in the proxy temperature may also be indicative of changes to the internal body temperature of the user. The proxy temperature is determined, at least in part, by comparing differences between the first temperature data and the second temperature data. If the first temperature data is substantially similar to the second temperature data (within a temperature threshold), then data from each of the sensors is utilized to determine an average temperature to serve as the proxy temperature. However, if information is different, then various techniques may be utilized, such as collecting an average over time, finding a correlation between min and max temperatures, and the like.

As noted herein, the proxy temperature may be an estimate or calculation of an internal body temperature for the user. The proxy temperature may be determined using one or more data analysis techniques that may correlate at least one of the first temperature data or the second temperature data to a body temperature. As an example, if a user is sleeping and has the wearable computing device under the covers with them, that environment may enable a correlation to be drawn between the proxy temperature and the body temperature because the environment may create a steady state or closed systems for measurements. It should be appreciated that other sensor data may also be used to determine whether such a state exists, such as a UV or light detector or a proximity detector, among other sensors or components. Additionally, in various embodiments, data may be acquired over time in order to develop the correlation between skin temperature and/or internal device temperature and body temperature. For example, it may be determined that a chance in body temperature will also affect a change in the skin temperature or internal device temperature, and as a result, that change may also be monitored.

In various embodiments, as shown at 808, a temperature change, relative to the proxy temperature, is determined. For example, the proxy temperature may increase over a period of time, and as a result, the change in temperature would be indicative of a change over a baseline or over a previous period of time. As shown at 810, the change in temperature may also be evaluated against a temperature threshold. If the change exceeds the temperature threshold, then the change in temperature may be indicative of a physiological change, such as a fever, ovulation, or the like. If the change does not exceed the threshold, then the change may be recorded and, if the change is presented over time, may be utilized to update a user baseline.

A user may preferentially wish to wear several devices (e.g., one on each wrist) or even a separate proximal temperature patch in order to provide a more robust proxy for core body temperature. In such cases, temperature estimates can be combined via averaging or other techniques to provide a more robust estimate.

Furthermore, a skin temperature device embedded in a wearable can be set to augment information about a user's sleep stage and quality. It is known that thermoregulation is reduced during REM sleep, so that a higher degree of temperature variability will be seen during these stages. Skin temperature can also reflect overall thermal comfort of a user and enhance or disrupt sleep. The temperature patterns shown in FIG. 7 can be used by a user to correlate with personal sleep comfort.

Respiration rate, heart rate, and heart rate variability are some health metrics that are easily measured by consumer devices and which can potentially provide early signs of illness. This makes consumer devices a valuable tool for detection of various illnesses and, in accordance with embodiments of the present disclosure, prediction of illnesses or severity based on data acquired from consumer devices.

Embodiments of the present disclosure include one or more machine learning systems, which may include one or more logistic regression classifiers, to predict the need for hospitalization or illness severity of patients given the symptoms experienced, age, sex, and BMI, among other potential information. Furthermore, systems and methods may include a one or more of a Convolutional Neural Network classifier to predict whether a person is sick on any specific day given respiration rate, heart rate, and heart rate variability data for that day and for the preceding days. Embodiments of the present disclosure have identified that respiration rate and heart rate are typically elevated by illness, while heart rate variability is decreased. Measuring these metrics can help in early diagnosis, and in monitoring the progress of a disease. The symptoms presented may also be indicative of the severity of the disease. For example, certain illnesses may lead to more several outcomes based on demographics and co-morbidities, such as being male, being older, or having a high BMI.

The heart rate variability is often decreased in subjects who are exhibiting symptoms of illness, while the heart rate and respiration rate are often elevated. Recent studies have shown that measuring these metrics using a consumer smart watch or tracker device may assist in early illness detection, for example of respiratory illnesses. Embodiments of the present disclosure illustrate that user symptoms have a prognostic value for predicting onset of an illness and severity.

Respiratory illnesses and other highly transmittable diseases may lead to large numbers of people becoming ill and infecting others, further propagating the illness through a population. For example, the year 2020 has seen the emergence of a global pandemic caused by the Severe Acute Respiratory Syndrome Coronavirus 2 (SARS-CoV-2) virus (e.g., Covid-19). The disease caused by this virus typically presents as a lower respiratory infection, though many atypical presentations have been reported. This has caused a major health challenge globally due to the apparent high transmissibility of this virus in a previously unexposed population. Of particular concern is that the primary mechanisms by which the disease is transmitted are still somewhat under debate (e.g., the importance of airborne versus fomite transmission), and the potential for infection by asymptomatic and pre-symptomatic patients. The disease is highly contagious, with transmission possible 2.3 days prior to the onset of symptoms, and peaking 0.7 days prior to the onset of symptoms, as explained in He et al. As a result, early detection would be advantageous for SARS-CoV-2 and other highly transmitted illnesses.

The popularity and widespread availability of consumer wearable devices has made possible the use of health metrics such as respiration rate, heart rate, heart rate variability, sleep, steps, etc. in order to predict the onset of respiratory illnesses, such as SARS-CoV-2. For example, as Karjalaninen et al. has identified that a 1 °C rise in body temperature can increase heart rate by 8.5 beats per minute on average. Accordingly, measuring the resting heart rate, or heart rate during sleep, can therefore be a useful diagnostic tool. Similarly, the respiration rate is elevated when patients present with a fever, as shown in Jensen et al. Heart Rate Variability (HRV) is the variability in the time between successive heart beats (the time between successive heart beats is called the "RR interval"), and is a non-invasive probe of the autonomic nervous system (Shaffer et al.) and lowered values are indicative of increased mortality (Tsuji et al.) and may provide early diagnosis of infection (Ahmad et al.). As an example, a study of heart rate variability in critically ill Covid-19 patients showed that the approximate entropy and the sample entropy were decreased in Covid-19 patients compared to critically ill sepsis patients (Kamaleswaran et al.).

Zhu et al. studied heart rate and sleep data collected from Huami devices to potentially identify outbreaks of Covid-19. Menni et al. analyzed symptoms reported through a smart phone app and developed a model to predict the likelihood of Covid-19 based on the symptoms. Marinsek et al. studied data from Fitbit devices as a means for early detection and management of Covid-19. Miller et al. used the respiration rate obtained from Whoop devices to detect Covid-19. Mishra et al. analyzed heart rate, steps, and sleep data collected from Fitbit devices to identify the onset of Covid-19.

Embodiments of the present disclosure consider the correlation between changes in physiological signs related to respiration rate, heart rate and HRV, and the corresponding presence of diseases assessed both through confirmed laboratory testing and self-reported symptoms and the time-course of the disease. For example, wearable devices, such as those described herein, may include one or more sensors to measure heart rate and underlying interbeat intervals (RR) that characterize heart rate variability. Furthermore, the wearable devices may include user-facing applications that may provide prompts and/or receive input indicative of user responses. The user input may include information about potential symptoms as well as demographic data such as age, sex, body mass index, and relevant background medical information such as underlying conditions such as diabetes, coronary arterial disease, or hypertension.

As an example, the user device may be utilized to provide a survey to users for a particular or group of medical conditions. Continuing with the example related to Covid-19, but understanding that such survey questions could also be applicable to other conditions such as influenza, urinary tract infections, and the like, users may be asked whether they have been tested for the presence of a disease or illness, whether they had symptoms, and whether they were tested for any other disease or illness. This information may be collected to generate ground truth or training data for a machine learning system. For example, users with a confirmed positive test may then provide their symptoms, date of symptom onset, and the like, which may be correlated with information from their wearable device, to identify correlations between measured information and the potential symptoms. It should be appreciated as more users provide information, such as more information for confirmed positive cases, that the training data set may be expanded.

It should be appreciated that a variety of information may be utilized in order to provide a diagnostic or predictive tool. By way of example, demographic information may be utilized that evaluates user age, user sex, user geographic location, and the like. Furthermore, co-morbidities may also be provided and then utilized for further analysis, such as identifying co-morbidities with a higher likelihood of complications from a particular illness. Examples include hypertension, diabetes, coronary artery disease, asthma, stroke, chronic kidney disease, chronic lung disease, chronic liver disease, or any other illness or disease that a user may self-report.

Furthermore, various embodiments may ask users that choose to participate in the survey about their symptoms. It should be appreciated that, even if the user did not have symptoms (e.g., was asymptomatic) this information may provide valuable data where it is presented along with a confirmed positive diagnosis. As an example, with COVID-19, asymptomatic carriers are believed to be capable of spreading the illness. Accordingly, it may be valuable to identify other factors, such as information obtained from the sensors that may be indicative of infection when other symptoms would not otherwise prompt a user to receive a test. Furthermore, the severity of symptoms may be classified, such as "mild", "moderate", "severe", and "critical". It should be appreciated that such classification may be subjective, as one or more users may consider certain symptoms as more severe than others. Accordingly, in various embodiments, severity may also be classified by the treatment the user received in order to provide quantitative guidelines. As an example, the user may provide an answer to the type of treatment they received. A sample of responses is provided below in Table 1 with the associated severity.

| Response | Severity |
| --- | --- |
| I did not experience symptoms. | Asymptomatic |
| I self-treated alone. | Mild |
| I self-treated with someone's help. | Moderate |
| I required hospitalization without ventilation support. | Severe |
| I required ventilation. | Critical |
| Prefer not to say. | N/A |

Accordingly, the severity of the patient's symptoms may be grouped and analyzed, which may provide information to health officials in different regions. For example, different regions can identify percentages of their populations with different factors related to higher severity to prepare to treat patients in the event of high numbers of infections.

It should be appreciated that the information collected from users may be analyzed to identify a symptom prevalence, and in various embodiments, identify different numbers of symptoms that, when paired together and/or with different severities, may lead to a greater likelihood of hospitalization. As an example, a user with a confirmed positive test may have mild symptoms such as a cough and fatigue while other users that have a cough, fatigue, and shortness of breath typically have moderate or severe symptoms. In this manner, combinations of symptoms may also be utilized to predict severity of illness.

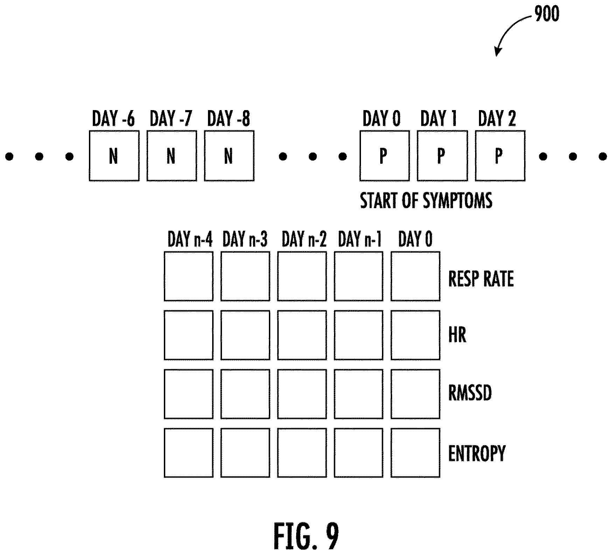

FIG. 9 is a representation 900 of a framework for assessing the presence of an illness according to the present disclosure. In this example, Day 0 represent the start of symptoms. A "Positive" class is assed to data drawn from the five days from Day 0 to Day 4 (shown as "P" in FIG. 9). These data are considered to be sick days. A "Negative" class is assigned to data obtained from the 8 days from Day -14 to Day -8 (shown as "N" in FIG. 9). The subject is considered "Healthy" on these days, with the appreciation that such a classification may be an assumption that is dependent on a variety of factors, such as number of days after infection that symptoms arise. The data from Day -7 to Day -1 is ignored since subjects may or may not show changes in their health metrics during this period, as noted above.

Embodiments of the present disclosure may calculation physiological data for each user on a daily basis, but it should be appreciated that other intervals of data collection may also be used. As an example, the data collection may include estimated mean respiration rate during deep (slow wave) sleep, estimated mean sleep rate during light sleep if deep sleep is insufficient, mean nocturnal heart rate during non-Rapid Eye Movement (NREM) sleep, Root Mean Square of Successive Differences (RMSSD) of the nocturnal RR series, and Shannon entropy of the nocturnal RR series. Further embodiments may include other estimates of heart rate variability such as spectral based parameters (referred to as VLF, LF, HF and LF/HF ratios in the literature), and other time-domain metrics such as SDNN, pNN50, TINN, detrended fluctuation analysis, and Allen variance. Given that the wearable can also measure the raw photoplethysmogram signal, physiological variables associated with blood pressure changes can be estimated. For example, the amplitude of the green PPG signal can be influence by the systolic pressure at a beat-by-beat level. A sequence of increasing PPG amplitudes can be interpreted as a sequence of increasing systolic pressures, and the corresponding RR intervals can be obtained. The sequence method can be used to form an estimate of the baroreflex sensitivity over a single night of data. Physiologists have shown that baroreflex sensitivity may reduce during periods of fever (Front Physiol. 2019; 10: 771. Published online 2019 Jun 25. doi: 10.3389/fphys.2019.00771). More generally, there is evidence that mean arterial pressure can increase during illness. The mean arterial pressure can be obtained from existing cuff based blood pressure readers, and entered manually on the wearable device, or imported into the associated set of data on the wearable app. Therefore, measurements of baroreflex sensitivity or mean arterial pressure can augment the set of values fed to a system for classification. Furthermore, another physiological metric of interest that can be obtained from a wearable is an estimate of the oxygen saturation in the blood ($SpO_2$). The $SpO_2$ can be obtained on a high resolution timescale (e.g., every second) and then summarized on a daily basis through metrics such as the mean $SpO_2$ over the night, the range of values displayed by the $SpO_2$ or even more sophisticated measurements of oxygen saturation variability (e.g., variance, spectral analysis, number of desaturations during the night).

Various embodiments may restrict or otherwise preferentially obtain data during the nighttime, such as the period midnight - 7 am. Selecting this period may minimize the effect of confounding effects such as exercise or caffeine intake. Moreover, data may be further restricted to particular times or conditions, such as when the users are still, which may be measured by an accelerometer or other device sensor. The RMSSD is a time domain measurement used to estimate vagally mediated changes (Shaffer et al.). In embodiments, it is computed in five-minute intervals and the median value of these individual measurements over the whole night is calculated. The Shannon entropy is a non-linear time domain measurement computed using the histogram of RR intervals over the entire night.

Using the symptoms as input features, embodiments train a logistic regression classifier to predict the need for hospitalization (e.g., categories that are "severe" and "critical"). Additionally, embodiments may identify the onset of illness by training a model. Since health metrics such as respiration rate, heart rate, heart rate variability, blood pressure, baroreflex sensitivity and oxygen saturation values can vary substantially between users, embodiments compute Z scored equivalents, represented by Equation (1),

$$Z_x = \frac{x - \mu_x}{\sigma_x} , \qquad \qquad \text{Equation (1)}$$

where x is a data from a sensor and/or calculated from a sensor, which may correspond to respiration rate, heart rate, RMSSD, or entropy and $\mu_x$ and $\sigma_x$ are the rolling mean and rolling standard deviation of the metric being measured.

In various embodiments, the rolling values of the mean and standard deviation are determined by evaluating the first seven days of data to obtain an initial estimate of mean and standard deviation. It should be appreciated that seven days is used as an example only and in various other embodiments different time periods may be used. For each additional day of data, data points are evaluated for outliers using Equation (1). Thereafter, a probably is computed from the Z score using a one-sided t-test (only positive values for respiration rate and heart rate, only negative values for RMSSD and entropy). If the p-value is smaller than a chosen threshold, the data point is considered to be anomalous (i.e., the subject is presumed sick) and ignored for the computation of $\mu_x$ and $\sigma_x$. Otherwise, the point is added to the list and an updated mean and standard deviation are computed. The threshold for the p-value may be particularly selected on a variety of factors. As will be understood, if the threshold is too low, there is a risk of including sick days in the computation of $\mu_x$ and $\sigma_x$. However, if the threshold is too high, the mean is biased lower or higher than the true value. As an example, the threshold may be set to 0.05, but such an example is provided for illustrative purposes only.

For any given day $D_n$, the mean and standard deviation are evaluated on the day $D_i$ closest to $D_n$, such that i < = n. In the event the subject has fewer than 7 days of data (or any other number selected), there is no calculation of the mean and standard deviation. In various embodiments, using Equation (1) and the estimated values of $\mu_x$ and $\sigma_x$, Z scores are calculated for the provided health metrics (e.g., sensor data and/or data derived from the sensors). In various embodiments, the Z scores are thresholded. In this example, $Z_{max}$ = +5 and $Z_{min}$ = -3, but it should be appreciated that other thresholds may be selected and rescaled to the range (0,1).

Data may then be evaluated by constructed a $4 \times 5$ matrix with normalized Z scores for each day $D_n$. In various embodiments, the Z-scores correspond to certain health metrics (in this example 4 health metrics), measured on the days $D_n$ ... $D_{n-4}$. Each day is represented by a matrix with that day's data along with the previous four days data. Missing data may be filled in using liner interpolation. In certain embodiments, it may be desirable to limit the amount of missing data allowed. For example, missing data may only be filled in if there is a threshold amount of data, such as a minimum of 3 days of data. Each matrix may be utilized to form an "image" by resizing each 4x5 matrix to a $28 \times 28 \times 1$ matrix, with the last dimension indicating that there is only one color channel.

It should be appreciated that, at an initial step, a machine learning system may be trained using a subset of training referred to as training data. For example, with 464 unique individuals with sufficient data, 4815 images may be obtained. This may be divided into a training set with 70% of the data, with the remainder divided into two hold-out sets. One hold-out set (the "CV set") may be used for cross-validation. Thereafter, a machine learning system, such as a convolutional neural network, may be trained. The network may include a single convolutional stage and a single dense stage with non-linearity introduced in the form of a "ReLu" layer, while the output layer is a softmax function.

By way of example, a logistic regression model may be trained to predict the need for hospitalization with four fold cross-validation, using the symptoms as input features, along with the age, sex, and BMI. The probability of the need for hospitalization p may be approximated by Equation (2),

$$z = \alpha + \sum_i w_i s_i$$

$$p = \frac{1}{1 + e^{-z}},$$ 

Equation (2)

where $\alpha = 3{:}62$, $s_i$ is a symptom (1 if the symptom is present, and 0 otherwise), and $w_i$ is the weight corresponding to symptom $s_i$. The weights may be particularly selected based on diagnostic information, which may be collected and adjusted over time. For example, for a novel disease, as new symptoms are identified, weights may be increased.

In various embodiments, variables (e.g., age and BMI) may be scaled as shown in Equation (3),

$$x_s = \frac{x - \mu_x}{\sigma_x},$$ 

Equation (3)

where $x_s$ is the scaled version and x stands for age or BMI. $\mu_x$ and $\sigma_x$ are the respective mean and standard deviation. For the sex, 1 indicates male and 0 indicates female. Various embodiments may include a trained convolutional neural network to predict whether an individual is sick on any specific day given the Z scores for respiration rate, heart rate, RMSSD, and entropy for that day and the preceding four days. The results of training the model are presented in Table 2 below.

| Fold | Number of filters in the conv. stage | Number of neurons in the dense layer | Filter size | Test set AUC | CV set AUC |
|------|------|------|------|------|------|
| 1 | 256 | 256 | $2 \times 2$ | 0.771 | 0.766 |
| 1 | 256 | 256 | $3 \times 3$ | 0.771 | 0.765 |
| 1 | 256 | 256 | $5 \times 5$ | 0.751 | 0.741 |
| 1 | 256 | 256 | $7 \times 7$ | 0.752 | 0.745 |

(b) Variation with train/cv/test split.

| Fold | Number of filters in the conv. stage | Number of neurons in dense layer | Filter size | Test set AUC | CV set AUC |
|------|------|------|------|------|------|
| 1 | 256 | 256 | $2 \times 2$ | 0.771 | 0.766 |
| 2 | 256 | 256 | $2 \times 2$ | 0.797 | 0.689 |
| 3 | 256 | 256 | $2 \times 2$ | 0.764 | 0.750 |
| 4 | 256 | 256 | $2 \times 2$ | 0.712 | 0.705 |

(c) Convolutional and dense layers.

| Fold | Number of filters in the conv. stage | Number of neurons in the dense layer | Filter size | Test set AUC | CV set AUC |
|---|---|---|---|---|---|
| 1 | 256 | 256 | 2 × 2 | 0.771 | 0.706 |
| 1 | 256 | 128 | 2 × 2 | 0.769 | 0.765 |
| 1 | 128 | 256 | 2 × 2 | 0.770 | 0.764 |
| 1 | 128 | 128 | 2 × 2 | 0.766 | 0.765 |

As shown, three hyper-parameters are varied: the number of filters in the convolutional stage, the number of neurons in the dense stage, and the filter size which is of the form $k \times k$. The results sub-table shows the AUC for different choices of k. As shown, smaller filter sizes perform slightly better than others. The middle sub-table shows the modeling performed on various folds of data. The data is randomly split into training and hold-out sets, but the split is performed four times using a different seed each time, to reduce the risk of outliers in influencing the results. The AUC varies from 0.71 - 0.80 which indicates that some individuals show only small changes in health metrics while others show a more measurable change. The bottom sub-table experiments with the number of filters and the number of neurons in the dense layer, with very little effect on the AUC.

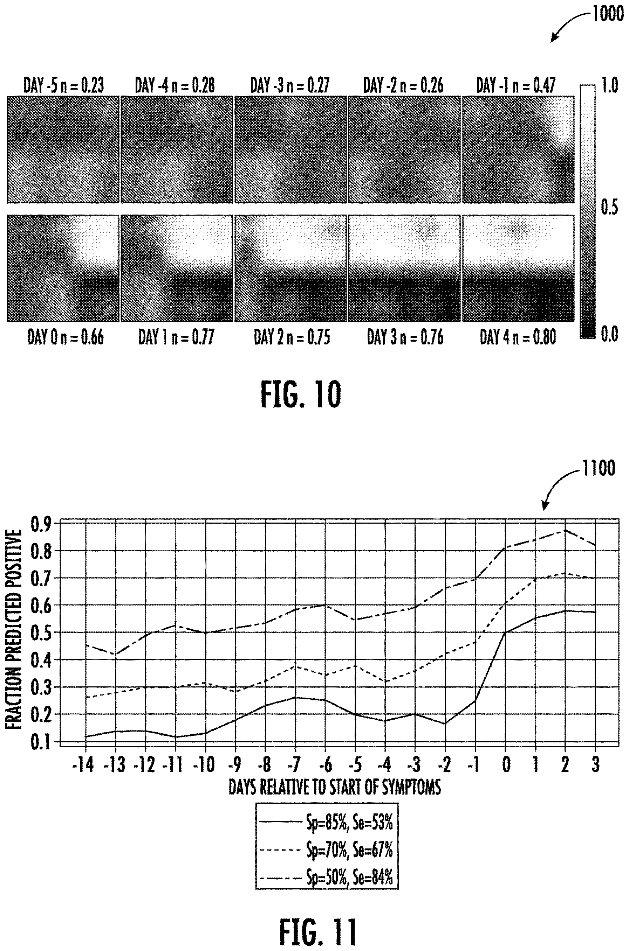

FIG. 10 illustrates an example input image for a single user according to the present disclosure. Shown are days from Day -5 to Day +4, where Day 0 represents the start of symptoms. As illustrated, the images from Day -5 to Day -2 show no prominent features and are consistent with normal health metrics. Day -1 shows a bright spot developing in the top right corner indicating an elevated respiration rate and heart rate, while the bottom right corner is dark implying a lower than normal RMSSD and entropy. This pattern evolves to a bright band and a dark band by Day 3 indicating significant changes to the health metrics sustained over many days. The probability score increases from Day -2 to Day -1 and remains high thereafter. A suitable threshold to classify the probability score into sick/ healthy classes is determined by the specificity/sensitivity requirements.

It should be appreciated that specificity/sensitivity may be adjusted based on changes to various parameters. For example, a true positive that is a prediction of sickness when the subject is sick is desirable. While a false positive, a prediction of sickness on days the subject is not sick, is less desirable. Even less desirable is a false negative, where the subject is sick but is predicted as not being sick. Table 3 below lists the values of specificity and sensitivity for a given prediction threshold (e.g., (for a specific train/ cv/ test split, i.e., Fold# 1) showing a 90% specificity with 48% sensitivity if the threshold is set to 0.533, or a 91% sensitivity with 31% specificity if the threshold is set to 0.286. A high specificity allows for a detection algorithm that does not cause a lot of false alarm, while a high sensitivity is preferred for an early warning system that encourages people to stay at home even if they are at low risk.

| Threshold | Specificity | Sensitivity |
|---|---|---|
| 0.603 | 0.954 | 0.381 |
| 0.533 | 0.901 | 0.477 |
| 0.49 | 0.849 | 0.534 |
| 0.466 | 0.798 | 0.584 |
| 0.443 | 0.752 | 0.612 |
| 0.412 | 0.704 | 0.673 |
| 0.396 | 0.654 | 0.795 |
| 0.37 | 0.599 | 0.769 |
| 0.347 | 0.55 | 0.804 |
| 0.325 | 0.502 | 0.843 |
| 0.313 | 0.467 | 0.861 |
| 0.295 | 0.395 | 0.883 |
| 0.286 | 0.368 | 0.907 |

EP 4 196 006 B1

(continued)

| Threshold | Specificity | Sensitivity |
|---|---|---|
| 0.262 | 0.309 | 0.922 |
| 0.251 | 0.265 | 0.95 |

FIG. 11 is a graphical representation 1100 of a fraction of users in a test set who predicted positive (e.g., predicted "sick" for different days). Day 0 is the start of symptoms. Negative numbers indicate days prior to the start of symptoms, while positive numbers are days following the start of symptoms. Shown are predictions for three different choices of specificity (Sp) and sensitivity (Se) from Table 3. As illustrated, and described above, the lower specificity results in a larger number of positive predictions prior to the onset of symptoms.

Embodiments provide a systems and methods for predicting a positive diagnosis for an illness and/or a likely severity (e.g., need for hospitalization) using information that may be obtained from a wearable along with information provided by a user. Additionally, embodiments enable detection of certain symptoms, or symptom groups that provide a higher likelihood of severe cases that may lead to hospitalization or other elevated care. Furthermore, demographic information and/or co-morbidities may also be identified to determine a higher likelihood of a user having severe symptoms. In various embodiments, data that may be acquired from a user device may be utilized in the predictions, such as respiration rate, heart rate, and heart rate variability. Furthermore, in embodiments, temperature may also be an indicator, which may use a proxy temperature as described above. Embodiments also include a trained convolutional neural network to predict illness on any specific day given health metrics for that day and the preceding four days. A high sensitivity model may detect illness 1-2 days early, while a high specificity model is less likely to produce faulty predictions of illness.

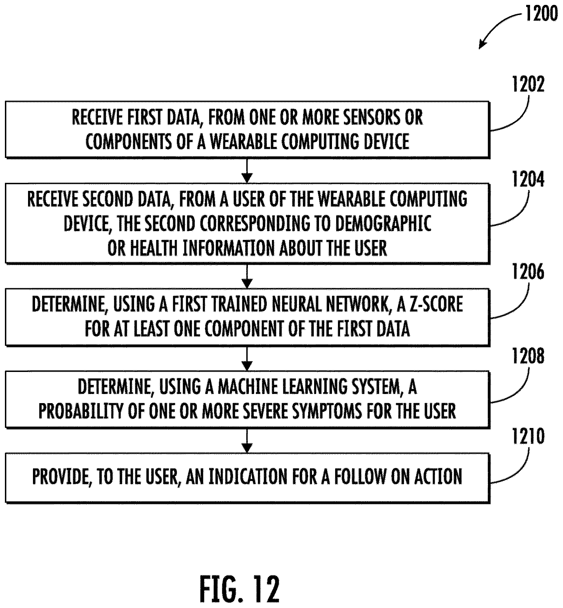

FIG. 12 is a flow chart of an embodiment of a method 1200 for predicting an illness and/or likely severity of an illness according to the present disclosure. In this example, as shown at 1202, first data is received from one or more sensors of a wearable computing device. As noted above, the sensors may provide data related to respiration rate, heart rate, and heart rate variability, temperature, blood pressure, oxygen saturation, and the like. Additionally, as shown at 1204, second data may be provided by the user related to demographic or health information. For example, demographic information may include age, sex, geographic location, etc. Also, health information may include BMI, co-morbidities, and/or symptoms. As shown at 1206, the information may be collected and processed, using a trained neural network, to develop a Z-score. In such embodiments, the Z-scores may be processed into an image matrix and then evaluated, over time, to identify one or more symptoms indicative of an illness on a particular day. In various embodiments, the data is provided over a series of days, with changes in various factors providing the indicators of illness. Furthermore, in various embodiments, as shown at 1208, the data may be processed using a machine learning system, such as a trained linear classifier to predict a severity of the symptoms and/or illness for the user. The severity may be related to a likelihood the user will need intervention, such as hospitalization, as a result of the illness. As shown at 1210, the wearable computing device may provide a prompt to the user for a follow on action. For example, the wearable computing device may identify likely symptoms prior to illness and provide information to the user to remain at home or to see medical attention. In this manner, illnesses may be predicted along with likely severity. Alternative embodiments of the symptom prediction have also been considered. For example, a technique implementing change point analysis to determine the point in time in which a person switches form healthy to ill can be considered (see Aminikhanghahi S, Cook DJ. A Survey of Methods for Time Series Change Point Detection. Knowl Inf Syst. 2017;51(2):339-367. doi:10.1007/s10115-016-0987-z for a survey of common methods used to determine change points).

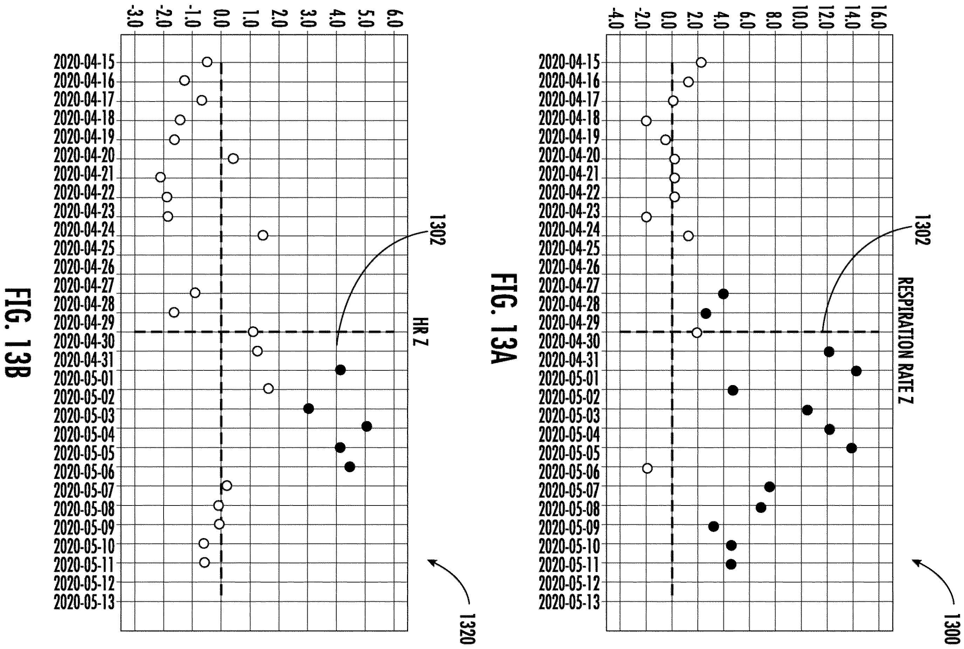

FIGS. 13A-13D are graphical representations of Z-scores associated with different data components for a hypothetical user having a respiratory illness according to the present disclosure. In various embodiments, FIGS. 13A-13D illustrate statistically significant changes associated with an onset of illness, which may be utilized to provide an alert to a user indicating illness onset or potential illness. It should be appreciated that the changes may be evaluated as a percentage change, absolute change, or the like. Furthermore, as shown in FIGS. 13A-13D, information may be graphically provided to users with different levels, such as color-coding to illustrate different variability ranges to enable a user to track their health over time.

FIG. 13A illustrates representative data from an individual user who was diagnosed with a respiratory illness, which may be COVID-19. In this example, a graphical representation 1300 of respiration rate Z-scores is presented, with the y-axis corresponding to the Z-score while the x-axis corresponds to a date. The illustrated representation 1300 includes a diagnosis date 1302 of April 28, 2020. As shown in the illustrated embodiment, the respiration rate Z-scores begin to increase before and after the diagnosis date 1302. This example includes an alert system with color-coding, where red does indicate a high statistical significance, yellow dots are moderate level of significance, and green dots are within normal variability ranges.

14

In certain embodiments, a threshold number of days at high statistic significant or moderate statistically significant may be used as an indicator for providing an alert to a user. For example, in this example, the user may be alerted on April 29, 2020, where three of the previous four days were of high statistical significance. It should be appreciated that a variety of different methods maybe used for establishing a threshold, such as increasing Z-scores over a number of days, increased in Z-score values, increase in average Z-score values over a time period, or any combination thereof. Additionally, it should be appreciated that the respiration rate Z-scores may be one factor in determining whether or not to provide an alert to the user and may, in various embodiments, but a weighted factor for determining whether a threshold amount of data has been acquired to make a suggestion to the user, such as to go and perform a diagnostic test, has been acquired.

FIGS. 13B-13D include additional representations 1320, 1340, 1360 corresponding to a nocturnal Z-scored heart rate, Z-scored RMSSD, and Z-scored Shannon entropy for deep sleep RR intervals. FIG. 13B also shows an increase after diagnosis 1302. Accordingly, in certain embodiments, the nocturnal Z-score heart rate may also be utilized to evaluate potential onset of illness and/or provide a recommendation for a diagnostic test. For example, the combination of both an increased Z-scored respiratory rate with an increased Z-scored heart rate may, in various embodiments, be sufficient to satisfy a threshold to provide a notification to the user.

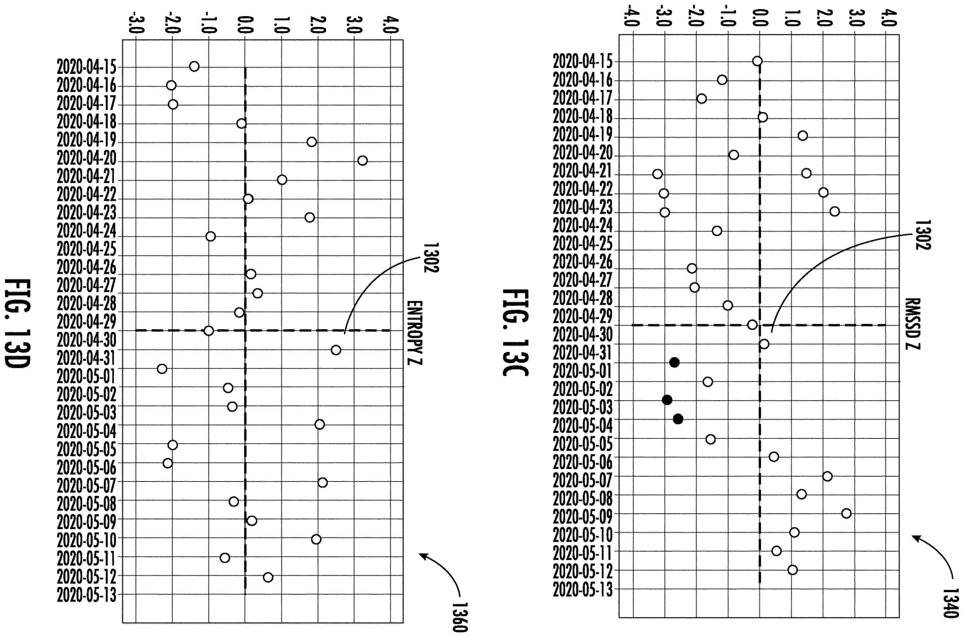

FIGS. 13C and 13D further illustrate statistically significant changes after diagnosis 1302 for the Z-scored RMSSD and the Z-scored Shannon entropy. For example, as shown in the representation 1340, three out of four days between April 30 and May 3 have a high statistical significance low Z-scored RMSSD. These dates correspond and/or partially overlap with the high statistical readings shown in the Z-scored respiratory rate and Z-scored heart rate. The representation 1360 of FIG. 13D also shows a medium statistical significance deviation on April 30. Accordingly, embodiments of the present disclosure may utilize one or more sets of data in order to determine various changes in the Z-score that may be indicative of illness onset.

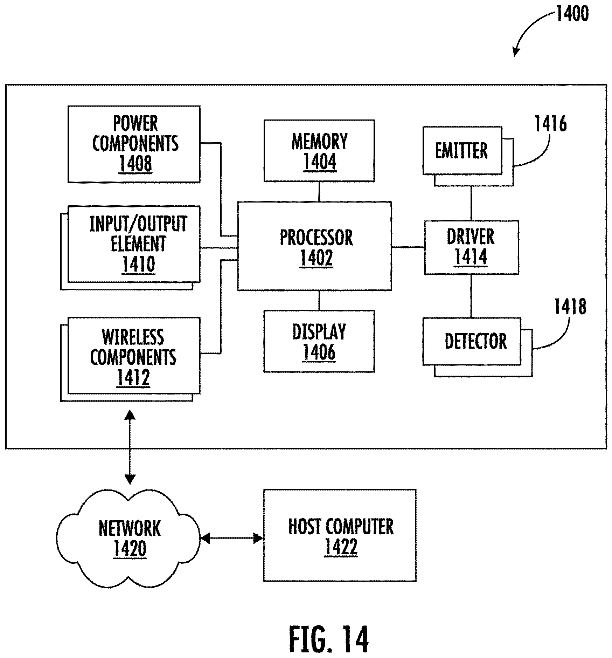

FIG. 14 illustrates a set of basic components 1400 of one or more devices of the present disclosure, in accordance with various embodiments of the present disclosure. In this example, the device includes at least one processor 1402 for executing instructions that can be stored in a memory device or element 1404. As would be apparent to one of ordinary skill in the art, the device can include many types of memory, data storage or computer-readable media, such as a first data storage for program instructions for execution by the processor(s) 1402, the same or separate storage can be used for images or data, a removable memory can be available for sharing information with other devices, and any number of communication approaches can be available for sharing with other devices. The device may also include, at least, as a display 1406 (e.g., a touch screen, electronic ink (e-ink), organic light emitting diode (OLED) or liquid crystal display (LCD)), one or more power components, an input/output element 1410, one or more wireless components 1412, an emitter 1416, a driver 1414, and/or a detector 1418, as well as devices such as servers that convey information via other means, such as through a system of lights and data transmissions. The device may also include a port, network interface card, or wireless transceiver that enables communication over at least one network 1420. The input/output device 1410 is able to receive conventional input from a user. This input/output device 1410 can include, for example, a push button, touch pad, touch screen, wheel, joystick, keyboard, mouse, trackball, keypad or any other such device or element whereby a user can input a command to the device. These input/output devices 1410 can also be connected by a wireless infrared or Bluetooth or other link as well in some embodiments. In some embodiments, however, such a device might not include any buttons at all and might be controlled only through a combination of visual and audio commands such that a user can control the device without having to be in contact with the device.

As discussed, different approaches can be implemented in various environments in accordance with the described embodiments. As will be appreciated, although a Web-based environment is used for purposes of explanation in several examples presented herein, different environments may be used, as appropriate, to implement various embodiments. The system includes an electronic client device, which can include any appropriate device operable to send and receive requests, messages or information over an appropriate network and convey information back to a user of the device. Examples of such client devices include personal computers, cell phones, handheld messaging devices, laptop computers, set-top boxes, personal data assistants, electronic book readers and the like. The network can include any appropriate network, including an intranet, the Internet, a cellular network, a local area network or any other such network or combination thereof. Components used for such a system can depend at least in part upon the type of network and/or environment selected. Protocols and components for communicating via such a network are well known and will not be discussed herein in detail. Communication over the network can be enabled via wired or wireless connections and combinations thereof. In this example, the network includes the Internet, as the environment includes a Web server for receiving requests and serving content in response thereto, although for other networks, an alternative device serving a similar purpose could be used, as would be apparent to one of ordinary skill in the art.

The illustrative environment includes at least one application server and a data store. It should be understood that there can be several application servers, layers, or other elements, processes, or components, which may be chained or otherwise configured, which can interact to perform tasks such as obtaining data from an appropriate data store. As used herein, the term "data store" refers to any device or combination of devices capable of storing, accessing, and retrieving data, which may include any combination and number of data servers, databases, data storage devices and data storage media, in any standard, distributed or clustered environment. The application server can include any appropriate hardware and software for integrating with the data store as needed to execute aspects of one or more applications for the client device and handling a majority of the data access and business logic for an application.

The application server provides access control services in cooperation with the data store and is able to generate content such as text, graphics, audio and/or video to be transferred to the user, which may be served to the user by the Web server in the form of HTML, XML, or another appropriate structured language in this example. The handling of all requests and responses, as well as the delivery of content between the client device and the application server, can be handled by the Web server. It should be understood that the Web and application servers are not required and are merely example components, as structured code discussed herein can be executed on any appropriate device or host machine as discussed elsewhere herein. The data store can include several separate data tables, databases or other data storage mechanisms and media for storing data relating to a particular aspect. For example, the data store illustrated includes mechanisms for storing content (e.g., production data) and user information, which can be used to serve content for the production side. The data store is also shown to include a mechanism for storing log or session data. It should be understood that there can be many other aspects that may need to be stored in the data store, such as page image information and access rights information, which can be stored in any of the above listed mechanisms as appropriate or in additional mechanisms in the data store. The data store is operable, through logic associated therewith, to receive instructions from the application server and obtain, update, or otherwise process data in response thereto. In one example, a user might submit a search request for a certain type of item. In this case, the data store might access the user information to verify the identity of the user and can access the catalog detail information to obtain information about items of that type. The information can then be returned to the user, such as in a results listing on a Web page that the user is able to view via a browser on the user device. Information for a particular item of interest can be viewed in a dedicated page or window of the browser.

Each server typically will include an operating system that provides executable program instructions for the general administration and operation of that server and typically will include computer-readable medium storing instructions that, when executed by a processor of the server, allow the server to perform its intended functions. Suitable implementations for the operating system and general functionality of the servers are known or commercially available and are readily implemented by persons having ordinary skill in the art, particularly in light of the disclosure herein.

The environment in one embodiment is a distributed computing environment utilizing several computer systems and components that are interconnected via communication links, using one or more computer networks or direct connections. However, it will be appreciated by those of ordinary skill in the art that such a system could operate equally well in a system having fewer or a greater number of components than are illustrated. Thus, the depiction of the systems herein should be taken as being illustrative in nature and not limiting to the scope of the disclosure.

The various embodiments can be further implemented in a wide variety of operating environments, which in some cases can include one or more user computers or computing devices, which can be used to operate any of a number of applications. User or client devices can include any of a number of general purpose personal computers, such as desktop or notebook computers running a standard operating system, as well as cellular, wireless, and handheld devices running mobile software and capable of supporting a number of networking and messaging protocols. Devices capable of generating events or requests can also include wearable computers (e.g., smart watches or glasses), VR headsets, Internet of Things (IoT) devices, voice command recognition systems, and the like. Such a system can also include a number of workstations running any of a variety of commercially-available operating systems and other known applications for purposes such as development and database management. These devices can also include other electronic devices, such as dummy terminals, thin-clients, gaming systems and other devices capable of communicating via a network.

Most embodiments utilize at least one network that would be familiar to those skilled in the art for supporting communications using any of a variety of commercially-available protocols, such as TCP/IP, FTP, UPnP, NFS, and CIFS. The network can be, for example, a local area network, a wide-area network, a virtual private network, the Internet, an intranet, an extranet, a public switched telephone network, an infrared network, a wireless network, and any combination thereof.

In embodiments utilizing a Web server, the Web server can run any of a variety of server or mid-tier applications, including HTTP servers, FTP servers, CGI servers, data servers, Java servers and business application servers. The server(s) may also be capable of executing programs or scripts in response requests from user devices, such as by executing one or more Web applications that may be implemented as one or more scripts or programs written in any programming language, such as Java®, C, C# or C++ or any scripting language, such as Perl, Python or TCL, as well as combinations thereof. The server(s) may also include database servers, including without limitation those commercially available from Oracle®, Microsoft®, Sybase® and IBM® as well as open-source servers such as MySQL, Postgres, SQLite, MongoDB, and any other server capable of storing, retrieving, and accessing structured or unstructured data. Database servers may include table-based servers, document-based servers, unstructured servers, relational servers, non-relational servers, or combinations of these and/or other database servers.

The environment can include a variety of data stores and other memory and storage media as discussed above. These can reside in a variety of locations, such as on a storage medium local to (and/or resident in) one or more of the computers or remote from any or all of the computers across the network. In a particular set of embodiments, the information may reside in a storage-area network (SAN) familiar to those skilled in the art. Similarly, any necessary files for performing the functions attributed to the computers, servers or other network devices may be stored locally and/or remotely, as appropriate. Where a system includes computerized devices, each such device can include hardware elements that may be electrically coupled via a bus, the elements including, for example, at least one central processing unit (CPU), at least one input device (e.g., a mouse, keyboard, controller, touch-sensitive display element or keypad) and at least one output device (e.g., a display device, printer, or speaker). Such a system may also include one or more storage devices, such as disk drives, optical storage devices and solid-state storage devices such as random access memory (RAM) or read-only memory (ROM), as well as removable media devices, memory cards, flash cards, etc.

Such devices can also include a computer-readable storage media reader, a communications device (e.g., a modem, a network card (wireless or wired), an infrared communication device) and working memory as described above. The computer-readable storage media reader can be connected with, or configured to receive, a computer-readable storage medium representing remote, local, fixed and/or removable storage devices as well as storage media for temporarily and/or more permanently containing, storing, transmitting, and retrieving computer-readable information. The system and various devices also typically will include a number of software applications, modules, services, or other elements located within at least one working memory device, including an operating system and application programs such as a client application or Web browser. It should be appreciated that alternate embodiments may have numerous variations from that described above. For example, customized hardware might also be used and/or particular elements might be implemented in hardware, software (including portable software, such as applets) or both. Further, connection to other computing devices such as network input/output devices may be employed.

Storage media and other non-transitory computer readable media for containing code, or portions of code, can include any appropriate media known or used in the art, such as but not limited to volatile and non-volatile, removable and non-removable media implemented in any method or technology for storage of information such as computer readable instructions, data structures, program modules or other data, including RAM, ROM, EEPROM, flash memory or other memory technology, CD-ROM, digital versatile disk (DVD) or other optical storage, magnetic cassettes, magnetic tape, magnetic disk storage or other magnetic storage devices or any other medium which can be used to store the desired information and which can be accessed by a system device. Based on the disclosure and teachings provided herein, a person of ordinary skill in the art will appreciate other ways and/or methods to implement the various embodiments.

While various embodiments of the invention have been described above, it should be understood that they have been presented by way of example only, and not by way of limitation. Likewise, the various diagrams may depict an example architectural or other configuration for the disclosure, which is done to aid in understanding the features and functionality that can be included in the disclosure. The disclosure is not restricted to the illustrated example architectures or configurations, but can be implemented using a variety of alternative architectures and configurations. Additionally, although the disclosure is described above in terms of various exemplary embodiments and implementations, it should be understood that the various features and functionality described in one or more of the individual embodiments are not limited in their applicability to the particular embodiment with which they are described. They instead can be applied, alone or in some combination, to one or more of the other embodiments of the disclosure, whether or not such embodiments are described, and whether or not such features are presented as being a part of a described embodiment. Thus, the breadth and scope of the present disclosure should not be limited by any of the above-described exemplary embodiments.

Unless otherwise defined, all terms (including technical and scientific terms) are to be given their ordinary and customary meaning to a person of ordinary skill in the art, and are not to be limited to a special or customized meaning unless expressly so defined herein. It should be noted that the use of particular terminology when describing certain features or aspects of the disclosure should not be taken to imply that the terminology is being re-defined herein to be restricted to include any specific characteristics of the features or aspects of the disclosure with which that terminology is associated. Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide exemplary instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular embodiment of the invention. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

Where a range of values is provided, it is understood that the upper and lower limit, and each intervening value between the upper and lower limit of the range is encompassed within the embodiments.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

**Claims**

1. A wearable computing device (102, 200, 302, 1400), comprising:

   one or more sensors;
   at least one processor (1402); and
   at least one memory device (1404) comprising instructions that, when executed by the at least one processor (1402), cause the wearable computing device (102, 200, 302, 1400) to:

   receive, from a first sensor on the wearable computing device (102, 200, 302, 1400), first temperature data relating to a first temperature measurement of the user (100);

receive, from a second sensor on the wearable computing device (102, 200, 302, 1400), second temperature data relating to a second temperature measurement of the user (100), the second sensor being in a different location from the first sensor;

determine, based at least in part on the first temperature data and the second temperature data, a proxy temperature, comprising comparing differences between the first temperature data and the second temperature data, wherein, if the first temperature data is similar, within a temperature threshold, to the second temperature data, then data from each of the sensors is utilized to determine an average temperature to serve as the proxy temperature;

determine a temperature change, based at least in part on the proxy temperature;

compare the temperature change to a temperature threshold;

determine a preliminary assessment of a medical condition for the user (100) based on the temperature change; and

generate and display, via a display (204, 1406), a recommendation for the user (100) based on the preliminary assessment,

wherein the first temperature data is a skin temperature of the user (100) of the wearable computing device (102, 200, 302, 1400) and the second temperature data is an internal temperature of the wearable computing device (102, 200, 302, 1400).

2. The wearable computing device (102, 200, 302, 1400) of claim 1, wherein the proxy temperature is correlated to a core body temperature of the user (100) of the wearable computing device (102, 200, 302, 1400).

3. The wearable computing device (102, 200, 302, 1400) of claim 1, wherein the temperature threshold is at least one of a standard deviation away from a baseline temperature or a specified temperature.

4. The wearable computing device (102, 200, 302, 1400) of claim 1, wherein the instructions further cause the at least one processor (1402) to:

receive third temperature data, corresponding to the proxy temperature over a period of time; and determine, based at least in part on the third temperature data, a baseline temperature.

5. The wearable computing device (102, 200, 302, 1400) of claim 1, wherein the instructions further cause the at least one processor (1402) to:
determine the preliminary assessment of the medical condition for the user (100) based on the temperature change using at least one machine learning algorithm.

6. The wearable computing device (102, 200, 302, 1400) of claim 1, wherein the medical condition comprises at least one of fever, illness, an ovulation event, or circadian rhythm fluctuations.

**Patentansprüche**

1. Tragbare Rechenvorrichtung (102, 200, 302, 1400), Folgendes umfassend:

einen oder mehrere Sensoren;
mindestens einen Prozessor (1402); und
mindestens eine Speichervorrichtung (1404), die Anweisungen umfasst, die bei Ausführung durch den mindestens einen Prozessor (1402) die tragbare Rechenvorrichtung (102, 200, 302, 1400) zu Folgendem veranlassen:

Empfangen, von einem ersten Sensor an der tragbaren Rechenvorrichtung (102, 200, 302, 1400), erster Temperaturdaten, die sich auf eine erste Temperaturmessung des Benutzers (100) beziehen;
Empfangen, von einem zweiten Sensor an der tragbaren Rechenvorrichtung (102, 200, 302, 1400), zweiter Temperaturdaten, die sich auf eine zweite Temperaturmessung des Benutzers (100) beziehen, wobei sich der zweite Sensor an einer anderen Stelle als der erste Sensor befindet;
Bestimmen, mindestens teilweise basierend auf den ersten Temperaturdaten und den zweiten Temperaturdaten, einer Proxy-Temperatur, umfassend Vergleichen von Unterschieden zwischen den ersten Temperaturdaten und den zweiten Temperaturdaten, wobei, wenn die ersten Temperaturdaten innerhalb eines Temperaturschwellenwerts den zweiten Temperaturdaten ähnlich sind, Daten von jedem der Sensoren verwendet werden, um eine durchschnittliche Temperatur zu bestimmen, die als die Proxy-Temperatur dient;

Bestimmen einer Temperaturänderung, die mindestens teilweise auf der Proxy-Temperatur basiert;

Vergleichen der Temperaturänderung mit einem Temperaturschwellenwert;

Bestimmen einer vorläufigen Bewertung eines medizinischen Zustands für den Benutzer (100) basierend auf der Temperaturänderung; und

Erstellen und Anzeigen einer Empfehlung an den Benutzer (100) über eine Anzeige (204, 1406) basierend auf der vorläufigen Beurteilung,

wobei die ersten Temperaturdaten eine Hauttemperatur des Benutzers (100) der tragbaren Rechenvorrichtung (102, 200, 302, 1400) sind und die zweiten Temperaturdaten eine Innentemperatur der tragbaren Rechenvorrichtung (102, 200, 302, 1400) sind.

2. Tragbare Rechenvorrichtung (102, 200, 302, 1400) nach Anspruch 1, wobei die Proxy-Temperatur mit einer Körperkerntemperatur des Benutzers (100) der tragbaren Rechenvorrichtung (102, 200, 302, 1400) korreliert ist.

3. Tragbare Rechenvorrichtung (102, 200, 302, 1400) nach Anspruch 1, wobei der Temperaturschwellenwert mindestens eine Standardabweichung von einer Grundtemperatur oder eine bestimmte Temperatur ist.

4. Tragbare Rechenvorrichtung (102, 200, 302, 1400) nach Anspruch 1, wobei die Anweisungen ferner den mindestens einen Prozessor (1402) zu Folgendem veranlassen:

Empfangen von dritten Temperaturdaten, die der Proxy-Temperatur über eine Zeitspanne entsprechen; und

Bestimmen einer Grundtemperatur mindestens teilweise basierend auf den dritten Temperaturdaten.

5. Tragbare Rechenvorrichtung (102, 200, 302, 1400) nach Anspruch 1, wobei die Anweisungen den mindestens einen Prozessor (1402) ferner zu Folgendem veranlassen:

Bestimmen der vorläufigen Bewertung des Gesundheitszustands für den Benutzer (100) basierend auf der Temperaturänderung unter Verwendung mindestens eines maschinellen Lernalgorithmus.

6. Tragbare Rechenvorrichtung (102, 200, 302, 1400) nach Anspruch 1, wobei der Gesundheitszustand mindestens eines von Fieber, Krankheit, einem Ovulationsereignis oder Schwankungen des zirkadianen Rhythmus umfasst.

## Revendications

1. Dispositif informatique portable (102, 200, 302, 1400), comprenant :

un ou plusieurs capteurs ;

au moins un processeur (1402) ; et

au moins un dispositif de mémoire (1404) comprenant des instructions qui, lorsqu'elles sont exécutées par l'au moins un processeur (1402), amènent le dispositif informatique portable (102, 200, 302, 1400) à :

recevoir, à partir d'un premier capteur sur le dispositif informatique portable (102, 200, 302, 1400), des premières données de température relatives à une première mesure de température de l'utilisateur (100) ;

recevoir, à partir d'un second capteur sur le dispositif informatique portable (102, 200, 302, 1400), des deuxièmes données de température relatives à une seconde mesure de température de l'utilisateur (100), le second capteur étant dans un emplacement différent du premier capteur ;

déterminer, sur la base au moins en partie des premières données de température et des deuxièmes données de température, une température proxy, comprenant la comparaison des différences entre les premières données de température et les deuxièmes données de température, dans lequel, si les premières données de température sont similaires, dans un seuil de température, aux deuxièmes données de température, les données provenant de chacun des capteurs sont ensuite utilisées pour déterminer une température moyenne devant servir de température proxy ;

déterminer un changement de température, sur la base au moins en partie de la température proxy ;

comparer le changement de température à un seuil de température ;

déterminer une évaluation préliminaire d'un état de santé pour l'utilisateur (100) sur la base du changement de température ; et

générer et afficher, par l'intermédiaire d'un écran d'affichage (204, 1406), une recommandation pour l'utilisateur (100) sur la base de l'évaluation préliminaire,

dans lequel les premières données de température sont une température cutanée de l'utilisateur (100) du dispositif informatique portable (102, 200, 302, 1400) et les deuxièmes données de température sont une température interne du dispositif informatique portable (102, 200, 302, 1400).

2. Dispositif informatique portable (102, 200, 302, 1400) selon la revendication 1, dans lequel la température proxy est corrélée à une température corporelle centrale de l'utilisateur (100) du dispositif informatique portable (102, 200, 302, 1400).

3. Dispositif informatique portable (102, 200, 302, 1400) selon la revendication 1, dans lequel le seuil de température est au moins l'un d'un écart type par rapport à une température de base ou d'une température spécifiée.

4. Dispositif informatique portable (102, 200, 302, 1400) selon la revendication 1, dans lequel les instructions amènent également l'au moins un processeur (1402) à :

recevoir des troisièmes données de température, correspondant à la température proxy sur une période de temps ; et

déterminer, sur la base au moins en partie des troisièmes données de température, une température de référence.

5. Dispositif informatique portable (102, 200, 302, 1400) selon la revendication 1, dans lequel les instructions amènent également l'au moins un processeur (1402) à :
déterminer l'évaluation préliminaire de l'état de santé pour l'utilisateur (100) sur la base du changement de température à l'aide d'au moins un algorithme d'apprentissage automatique.

6. Dispositif informatique portable (102, 200, 302, 1400) selon la revendication 1, dans lequel l'état médical comprend au moins l'un parmi de la fièvre, une maladie, un événement d'ovulation ou des fluctuations du rythme circadien.

FIG. 1

FIG. 2

FIG. 3

FIG. 4A

FIG. 4B

FIG. 4C

FIG. 4D

FIG. 5

FIG. 6

FIG. 7

800

802

RECEIVE FIRST TEMPERATURE DATA, FROM A FIRST SENSOR OR COMPONENT OF A WEARABLE COMPUTING DEVICE, RELATING TO A SKIN TEMPERATURE

804

RECEIVE SECOND TEMPERATURE DATA, FROM A SECOND SENSOR OR COMPONENT OF THE WEARABLE COMPUTING DEVICE, RELATING TO A SKIN TEMPERATURE

806

DETERMINE, BASED AT LEAST IN PART ON THE FIRST TEMPERATURE DATA AND THE SECOND TEMPERATURE DATA, A PROXY TEMPERATURE

808

DETERMINE A TEMPERATURE CHANGE, BASED AT LEAST IN PART ON THE PROXY TEMPERATURE

810

DETERMINE THE TEMPERATURE CHANGE EXCEEDS A THRESHOLD

FIG. 8

900

| | DAY -6 | DAY -7 | DAY -8 | | | | DAY 0 | DAY 1 | DAY 2 |
|---|---|---|---|---|---|---|---|---|---|
| • • • | N | N | N | • • • | P | P | P | • • • |

START OF SYMPTOMS

| DAY n-4 | DAY n-3 | DAY n-2 | DAY n-1 | DAY 0 | |
|---|---|---|---|---|---|
| | | | | | RESP RATE |
| | | | | | HR |
| | | | | | RMSSD |
| | | | | | ENTROPY |

# FIG. 9

FIG. 10

FIG. 11

1200

1202

RECEIVE FIRST DATA, FROM ONE OR MORE SENSORS OR COMPONENTS OF A WEARABLE COMPUTING DEVICE

1204

RECEIVE SECOND DATA, FROM A USER OF THE WEARABLE COMPUTING DEVICE, THE SECOND CORRESPONDING TO DEMOGRAPHIC OR HEALTH INFORMATION ABOUT THE USER

1206

DETERMINE, USING A FIRST TRAINED NEURAL NETWORK, A Z-SCORE FOR AT LEAST ONE COMPONENT OF THE FIRST DATA

1208

DETERMINE, USING A MACHINE LEARNING SYSTEM, A PROBABILITY OF ONE OR MORE SEVERE SYMPTOMS FOR THE USER

1210

PROVIDE, TO THE USER, AN INDICATION FOR A FOLLOW ON ACTION

FIG. 12

FIG. 13A

FIG. 13B

FIG. 13C

FIG. 13D

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63065254
- WO 2019084312 A1
- US 2016331244 A1

### Non-patent literature cited in the description

- *Front Physiol.*, 25 June 2019, vol. 10, 771
- **AMINIKHANGHAHI S ; COOK DJ.** A Survey of Methods for Time Series Change Point Detection. *Knowl Inf Syst.*, 2017, vol. 51 (2), 339-367